# EUROPEAN PATENT SPECIFICATION

(11) **EP 3 427 653 B1**
(45) Date of publication and mention of the grant of the patent: **31.03.2021**
(21) Application number: 17782515.5
(22) Date of filing: 14.04.2017
(51) Int. Cl.: A61B 5/021, A61B 5/022, A61B 5/08, A61B 5/11, A61B 5/145, A61B 5/00

(54) **BIOLOGICAL INFORMATION ANALYSIS DEVICE, SYSTEM, AND PROGRAM**
VORRICHTUNG, SYSTEM UND PROGRAMM ZUR ANALYSE BIOLOGISCHER INFORMATIONEN
DISPOSITIF ET SYSTÈME D'ANALYSE D'INFORMATION BIOLOGIQUE ET PROGRAMME ASSOCIÉ

(30) Priority: 15.04.2016 JP 2016082463
(43) Date of publication of application: 16.01.2019
(73) Proprietor: Omron Corporation, Kyoto-shi, Kyoto 600-8530 (JP); Omron Healthcare Co., Ltd., Muko-shi, Kyoto 617-0002 (JP)
(72) Inventor: NAKAJIMA, Hiroshi, Kyoto-shi Kyoto 600-8530 (JP); WADA, Hirotaka, Kyoto-shi Kyoto 600-8530 (JP); TSUCHIYA, Naoki, Kyoto-shi Kyoto 600-8530 (JP); KASAI, Masaaki, Kyoto-shi Kyoto 600-8530 (JP); KAN, Eriko, Kyoto-shi Kyoto 600-8530 (JP); UENOYAMA, Toru, Tokyo 108-0075 (JP); OBAYASHI, Keiichi, Kyoto-shi Kyoto 600-8530 (JP); KOKUBO, Ayako, Kyoto-shi Kyoto 600-8530 (JP); OTA, Yuya, Kyoto-shi Kyoto 600-8530 (JP); SHIGA, Toshikazu, Muko-shi Kyoto 617-0002 (JP); KUWABARA, Mitsuo, Muko-shi Kyoto 617-0002 (JP); SATO, Hironori, Muko-shi Kyoto 617-0002 (JP); MIYAGAWA, Ken, Muko-shi Kyoto 617-0002 (JP); TSUTSUMI, Masakazu, Muko-shi Kyoto 617-0002 (JP)
(74) Representative: Isarpatent
(86) International application number: PCT/JP2017/015283
(87) International publication number: WO 2017/179702

(56) References cited:
- WO-A1-2016/017579
- JP-A- 2010 200 901
- JP-A- 2012 205 673
- US-A1- 2009 227 425
- US-A1- 2011 152 651
- US-A1- 2012 125 337

## Description

### TECHNICAL FIELD

The present invention relates to technology for acquiring useful information from a blood pressure waveform that has been measured.

### RELATED ART

There is a known technology for measuring changes in the internal pressure of a radial artery and recording the shape of a pressure pulse wave (blood pressure waveform). JP 2008-61824A discloses that a blood pressure waveform is measured using a tonometry method, and pieces of information such as an AI (Augmentation Index) value, a pulse wave period, a baseline fluctuation rate, sharpness, and an ET (Ejection Time) are acquired from the blood pressure waveform. Also, JP 2005-532111A discloses that a blood pressure waveform is measured using a wristwatch-type blood pressure meter, in which a mean arterial pressure, a mean systolic pressure, a mean diastolic pressure, a mean systolic pressure indicator, and a mean diastolic pressure indicator are calculated from the blood pressure waveform, and an alert is output when any of these values deviates from a reference value.

Further, US 2012/0125337 A1 discloses simultaneously monitoring a blood pressure waveform and a respiratory waveform in order to investigate sleep of a person. US 2011/0152651 A1 describes measuring a blood pressure waveform to determine indices of volume status of a patient. US 2009/0227425 A1 describes a respiration training machine.

### SUMMARY OF THE INVENTION

### PROBLEM TO BE SOLVED BY THE INVENTION

It is known that blood pressure can be controlled by using a breathing technique. However, it is envisioned that the amount of change in blood pressure varies for each person due to the influence of the characteristics and the disease state of each person, even if the same breathing pattern is used. Therefore, a standard routine for improvement in breathing does not have a sufficient blood pressure control effect. Also, for example, when a user experiences an increase in blood pressure or poor physical condition, even if the user knows that blood pressure can be controlled by using a breathing technique, the user does not know what specific measures can be taken.

The inventors of the present invention have worked hard to develop a blood pressure measurement device that can accurately measure an ambulatory blood pressure waveform for each heartbeat, and to put such a device into practical use. Through experiments performed on subjects during the development phase, the inventors have found that it can be possible to quantitatively evaluate a relationship between breathing and changes in blood pressure by accurately and non-invasively monitoring ambulatory blood pressure waveforms for each heartbeat.

Therefore, the present invention aims to provide a novel technology for acquiring information regarding a relationship between breathing and changes in blood pressure.

### MEANS FOR SOLVING THE PROBLEMS

To achieve the above-described aim, the present invention provides a biological information analysis device according to claim 1, a biological information analysis system according to claim 6, a program according to claim 8, and a method according to claim 9.

A biological information analysis device according to the present invention is a biological information analysis device that includes: an indicator extraction unit configured to extract, from time-series data regarding blood pressure waveforms consecutively measured by a sensor that is configured to be worn on a body part of a user and to be capable of non-invasively measuring a blood pressure waveform for each heartbeat, and from time-series data regarding breathing measured by a respiration sensor during a period of time corresponding to the time-series data regarding blood pressure waveforms, an indicator indicating a relationship between the user's breathing and changes in blood pressure; and a processing unit configured to perform processing that is based on the indicator thus extracted.

With the configuration according to the present invention, analysis is performed using time-series data regarding blood pressure waveforms measured from each heartbeat of the user, and time-series data regarding breathing measured during a period corresponding thereto. Therefore, it is possible to quantitatively analyze a relationship (a cause-effect relationship or a correlation) between breathing and change in blood pressure, which is a characteristic that is unique to the user.

Further, the indicator extraction unit is configured to specify a plurality of periods of time that have the same breathing pattern, from the time-series data regarding breathing, and extract an indicator indicating the relationship between breathing and changes in blood pressure for the breathing pattern, from pieces of data regarding blood pressure waveforms respectively corresponding to the plurality of periods of time. By analyzing pieces of data regarding blood pressure waveforms corresponding to periods of time that have the same breathing pattern, it is possible to improve the reliability and objectivity of the indicator. For example, it is preferable that the indicator extraction unit is configured to extract, as the indicator, a trend in changes in blood pressure and/or the amount of changes that is/are common to pieces of data regarding blood pressure waveforms.

According to the invention, the indicator extraction unit is configured to classify ways the user breathes, into a plurality of breathing patterns, based on the time-series data regarding the user's breathing. By classifying the user's breathing into a plurality of breathing patterns based on time-series data regarding the user's breathing, it is possible to acquire breathing patterns that are suitable for the user's characteristics.

For example, it is preferable that the time-series data regarding breathing includes, for each of a plurality of periods of time that include an exhalation period and an inhalation period, the length of the period of time and/or the amount of breath. If this is the case, the indicator extraction unit is configured to classify the plurality of periods of time into a plurality of breathing patterns based on the length of the period of time and/or the amount of breath. This is because the influence on blood pressure varies depending on the length of time spent for exhaling and inhaling and the amount of breath that is exhaled and inhaled.

According to the invention, the indicator extraction unit is further configured to extract an indicator indicating the relationship between breathing and changes in blood pressure for each of the plurality of breathing patterns. By identifying the relationship between breathing and changes in blood pressure for each of the plurality of breathing patterns, it is possible to acquire information that is very useful for knowing characteristics of the user's respiratory and circulatory organs.

For example, it is preferable that the processing unit is configured to perform processing to output information representing the relationship between the user's breathing and changes in blood pressure, based on the indicator. Because such information is provided, the user can understand the relationship between his/her breathing and changes in blood pressure, and recognize an effective way of breathing when regulating blood pressure.

According to the invention, the indicator extraction unit is configured to generate, for each of the plurality of breathing patterns, a relationship table that defines an indicator indicating the relationship between breathing and changes in blood pressure, and the processing unit is configured to select a breathing pattern that is to be followed in order to achieve a desired change in blood pressure, based on the relationship table, and recommend the selected breathing pattern to the user. With this configuration, it is possible to suggest an appropriate breathing pattern according to the user's characteristics and aim. Therefore, the user can effectively control his/her blood pressure by using a breathing technique.

It is preferable that the indicator extraction unit includes, as changes in blood pressure, at least one of: changes in systolic blood pressure; changes in diastolic blood pressure; changes in an AI (Augmentation Index); changes in the number of times a surge in blood pressure occurs; and changes in the amount of an increase in a surge in blood pressure.

The present invention can also be embodied as a biological information analysis method as defined in claim 9, or a program that causes a computer to function as the indicator extraction unit and the processing unit described above.

### EFFECTS OF THE INVENTION

According to the present invention, it is possible to provide a novel technology for acquiring information regarding a relationship between breathing and changes in blood pressure.

### BRIEF DESCRIPTION OF THE DRAWINGS

FIG. 1 shows a schematic external configuration of a biological information analysis system 10.
FIG. 2 is a block diagram showing a hardware configuration of the biological information analysis system 10.
FIG. 3 is a cross-sectional view schematically showing a configuration of a blood pressure measurement unit 20 and a state in which measurement is performed.
FIG. 4 shows a blood pressure waveform that is measured by the blood pressure measurement unit 20.
FIG. 5 is a block diagram illustrating processing that is performed by a biological information analysis device 1.
FIG. 6 shows a waveform (a blood pressure waveform) of a pressure pulse wave from a radial artery corresponding to one heartbeat.
FIG. 7 is a flowchart for processing that is performed to represent a relationship between breathing and changes in blood pressure as an indicator according to Example 1.
FIG. 8 is a diagram illustrating analysis of the relationship between breathing and changes in blood pressure according to Example 1.
FIGS 9A and 9B are diagrams showing an example of a relationship table that shows the relationship between breathing and changes in blood pressure according to Example 1.

### EMBODIMENTS OF THE INVENTION

The following describes a preferred embodiment of the present invention with reference to the drawings. Note that the following descriptions of components may be modified as appropriate depending on the configuration of a device to which the present invention is applied, and on various conditions, and the scope of the present invention is not intended to be limited to the following descriptions.

### Biological Information Analysis System

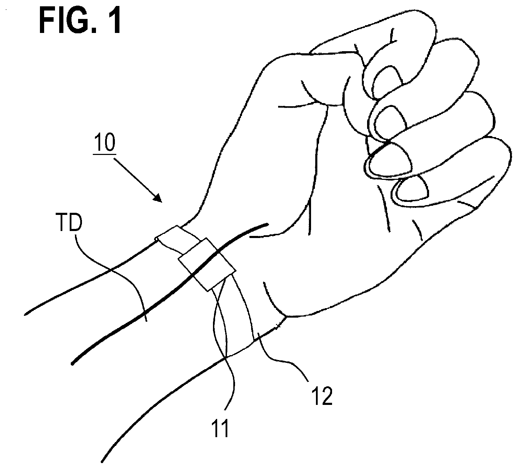

FIG. 1 shows a schematic external configuration of a biological information analysis system 10 according to an embodiment of the present invention. FIG. 1 shows a state in which the biological information analysis system 10 is worn on the left wrist. The biological information analysis system 10 includes a main body 11 and a belt 12 that is fixed to the main body 11. The biological information analysis system 10 is a so-called wearable device, and is worn such that the main body 11 is in contact with the skin on the palm side of the wrist, and the main body 11 is located over a radial artery TD that lies beneath the skin. Although the device is configured to be worn on the radial artery TD in the present embodiment, the device may be configured to be worn on another superficial artery.

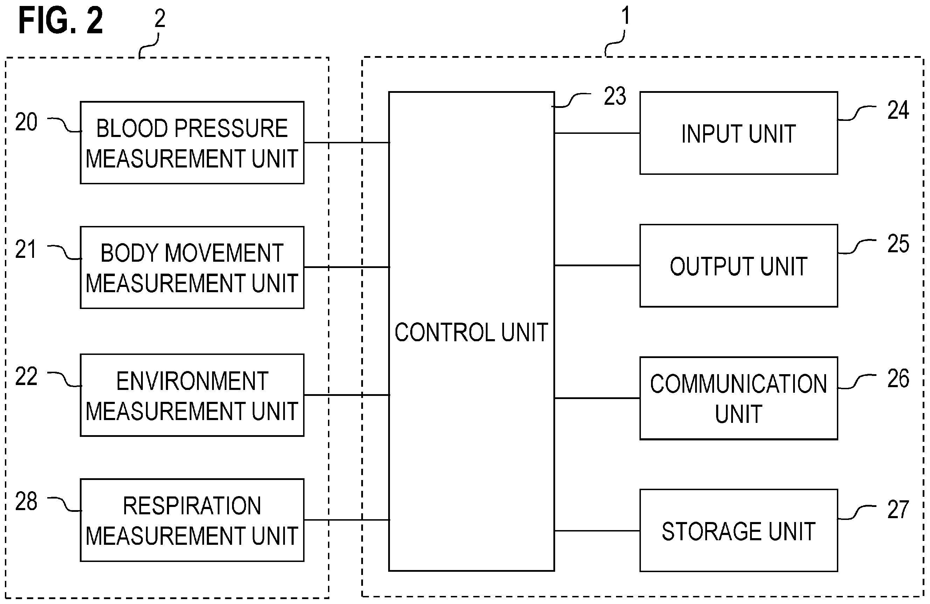

FIG. 2 is a block diagram showing a hardware configuration of the biological information analysis system 10. In general, the biological information analysis system 10 includes a measurement unit 2 and the biological information analysis device 1. The measurement unit 2 is a device that performs measurement to acquire information that is used to analyze biological information, and includes a blood pressure measurement unit 20, a body movement measurement unit 21, an environment measurement unit 22, and a respiration measurement unit 28. However, note that the configuration of the measurement unit 2 is not limited to that shown in FIG. 2. For example, a unit that measures biological information other than blood pressure or a body movement (e.g. body temperature, blood-sugar level, or brain waves) may be added. Also, any unit that is not used in the example described below is not an essential component, and may be omitted from the biological information analysis system 10. The biological information analysis device 1 is a device that analyzes biological information based on information acquired from the measurement unit 2, and includes a control unit 23, an input unit 24, an output unit 25, a communication unit 26, and a storage unit 27. The units 20 to 27 are connected to each other so that signals can be exchanged between them via a local bus or other signal lines. The biological information analysis system 10 also includes a power supply (a battery), which is not shown.

The blood pressure measurement unit 20 measures a pressure pulse wave from the radial artery TD by using a tonometry method. The tonometry method is for forming a flat area in the artery TD by pressing the artery from the skin with appropriate pressure, adjusting the balance between the internal pressure and the external pressure of the artery, and non-invasively measuring the pressure pulse wave using a pressure sensor.

The body movement measurement unit 21 includes a tri-axis acceleration sensor, and measures the movement of the user's body (body movement) using this sensor. The body movement measurement unit 21 may include a circuit that converts the format of an output from the tri-axis acceleration sensor into a format that is readable to the control unit 23.

The environment measurement unit 22 measures environmental information that may affect mental and physical conditions of the user (in particular the blood pressure). The environment measurement unit 22 may include, for example, an atmospheric temperature sensor, a humidity sensor, an illuminance sensor, an altitude sensor, a position sensor, and so on. The environment measurement unit 22 may include a circuit that converts the format of outputs from these sensors and so on into a format that is readable to the control unit 23.

The respiration measurement unit 28 is a unit that measures the state of the user's breathing. The respiration measurement unit 28 may include, for example, a respiration sensor such as a flow sensor. The respiration measurement unit 28 can at least discern between exhalation and inhalation, and measure the duration of exhalation and the duration of inhalation, and preferably, it can also measure the amount of breath. The respiration measurement unit 28 may include a circuit that converts the format of outputs from the respiration sensor and so on into a format that is readable to the control unit 23.

The control unit 23 performs various kinds of processing, such as controlling each unit of the biological information analysis system 10, acquiring data from the measurement unit 2, storing the acquired data in the storage unit 27, processing and analyzing data, and inputting and outputting data. The control unit 23 includes a hardware processor (hereinafter referred to as the "CPU") a ROM (Read Only Memory), a RAM (Random Access Memory), and so on. Processing that is performed by the control unit 23, which will be described later, is realized by the CPU reading and executing a program stored in the ROM or the storage unit 27. The RAM functions as a work memory that is used by the control unit 23 when performing various kinds of processing. Although acquisition of data from the measurement unit 2 and the storing of data in the storage unit 27 are performed by the control unit 23 in the present embodiment, it is possible to employ a configuration in which the measurement unit 2 directly stores (writes) data in the storage unit 27.

Each of the constituent components of the embodiment such as a measurement unit, an indicator extraction unit, a processing unit, a determination unit, a risk database, an input unit, an output unit, a case database, and so on may be implemented as pieces of hardware in the biological information analysis system 10. The indicator extraction unit, the processing unit, and the determination unit may receive an executable program stored in the storage unit 27, and execute the program. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as required. Databases such as the risk database and the case database may be implemented using the storage unit 27 and so on, and store pieces of information that are arranged such that a data search and data accumulation can be easily performed. Here, for example, the configuration, operations, and so on of the biological information analysis system 10 are disclosed in JP 2016-082069A. Also, the configuration, operations, and so on of the blood pressure measurement unit are disclosed in JP 2016-087003A.

The input unit 24 provides an operation interface for the user. For example, an operation button, a switch, a touch panel, and so on may be used.

The output unit 25 provides an interface that outputs information to the user. For example, a display device (such as a liquid crystal display) that outputs information using images, an audio output device or a beeper that outputs information using audio, an LED that outputs information by blinking, a vibration device that outputs information by vibrating, and so on may be used.

The communication unit 26 performs data communication with another device. Any data communication method such as a wireless LAN or Bluetooth (registered trademark) may be used.

The storage unit 27 is a storage medium that can store data and from which data can be read out, and stores programs that are to be executed by the control unit 23, pieces of measurement data acquired from the measurement units, and various kinds of data acquired by processing the pieces of measurement data, and so on. The storage unit 27 is a medium that accumulates pieces of information that are to be stored, through an electrical, magnetic, optical, mechanical, or chemical action. For example, a flash memory is used. The storage unit 27 may be a portable unit such as a memory card, or built into the biological information analysis system 10.

At least one unit or all units out of the body movement measurement unit 21, environment measurement unit 22, the control unit 23, the input unit 24, the output unit 25, and the storage unit 27 may be configured as a device that is separate from the main body 11. That is, as long as the blood pressure measurement unit 20 and the main body 11 that incorporates a circuit that controls the blood pressure measurement unit 20 are configured to be wearable on a wrist, the configurations of other units can be freely designed. If this is the case, the main body 11 cooperates with another unit via the communication unit 26. Various configurations can be conceived of. For example, the functions of the control unit 23, the input unit 24, and the output unit 25 may be realized using a smartphone application, and required data may be acquired from an activity monitor that has the functions of the body movement measurement unit 21 and the environment measurement unit 22. Also, a sensor that measures biological information other than blood pressure may be provided. For example, a sleep sensor, a pulse oximeter (SpO₂ sensor), a blood-sugar level sensor, and the like may be combined.

Although the sensor (the blood pressure measurement unit 20) that measures blood pressure and the component (including the control unit 23 and so on) that performs processing to analyze blood pressure waveform data are provided in one device in the present embodiment, they may be provided in separate members. In the present embodiment, the component (including the control unit 23 and so on) that performs processing to analyze biological information is referred to as a biological information analysis device, and the device that includes the combination of the measurement unit and the biological information analysis device is referred to as a biological information analysis system. However, these names are given for descriptive purposes, and the measurement unit and the component that performs processing to analyze biological information may be referred to as a biological information analysis device as a whole, or other names may be used.

### Measurement of Blood Pressure Waveform

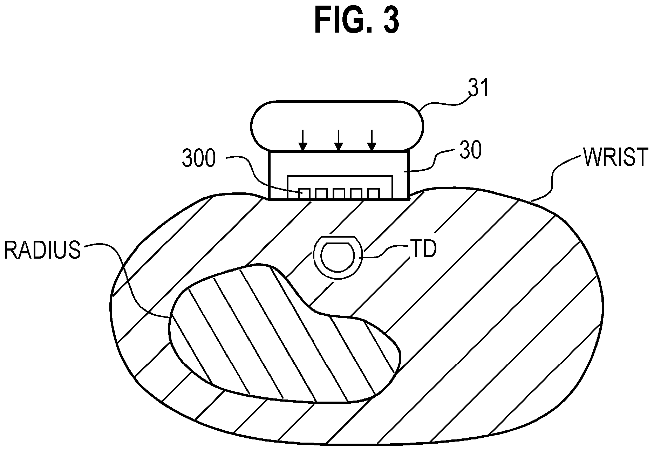

FIG. 3 is a cross-sectional view schematically showing the configuration of the blood pressure measurement unit 20 and a state in which measurement is performed. The blood pressure measurement unit 20 includes a pressure sensor 30 and a pressurizing mechanism 31 for pressing the pressure sensor 30 against a wrist. The pressure sensor 30 includes a plurality of pressure detection elements 300. The pressure detection elements 300 detect pressure and convert the pressure into an electrical signal. For example, elements that utilize a piezoresistive effect may be preferably used. The pressurizing mechanism 31 includes, for example, an air bag and a pump that adjusts the internal pressure of the air bag. As a result of the control unit 23 controlling the pump to increase the internal pressure of the air bag, the air bag expands and the pressure sensor 30 is pressed against the surface of the skin. Note that the pressurizing mechanism 31 may be any mechanism as long as it can adjust the pressing force of the pressure sensor 30 applied to the surface of the skin, and is not limited to a mechanism that uses an air bag.

Upon the biological information analysis system 10 being worn on a wrist and activated, the control unit 23 controls the pressurizing mechanism 31 of the blood pressure measurement unit 20 to keep the pressing force of the pressure sensor 30 in an appropriate state (a tonometry state). Then, pressure signals detected by the pressure sensor 30 are sequentially acquired by the control unit 23. Pressure signals acquired from the pressure sensor 30 are generated by digitizing analogue physical amounts (e.g. voltage values) output by the pressure detection elements 300, through an A/D converter circuit or the like that employs a well-known technology. Preferable analogue values such as current values or resistance values may be employed as the analogue physical amounts, depending on the type of the pressure detection elements 300. Signal processing such as the aforementioned A/D conversion may be performed using a predetermined circuit provided in the blood pressure measurement unit 20, or performed by another unit (not shown) provided between the blood pressure measurement unit 20 and the control unit 23. Each pressure signal acquired by the control unit 23 corresponds to an instantaneous value of the internal pressure of the radial artery TD. Therefore, it is possible to acquire time-series data regarding blood pressure waveforms by acquiring pressure signals with time granularity and continuity that make it possible to ascertain a blood pressure waveform for each heartbeat. The control unit 23 stores the pressure signals sequentially acquired from the pressure sensor 30, in the storage unit 27, together with information regarding points in time at which the pressure signals were measured. The control unit 23 may store the acquired pressure signals in the storage unit 27 without change, or store the pressure signals in the storage unit 27 after performing required signal processing on the pressure signals. Required signal processing includes, for example, processing that is performed to calibrate each pressure signal such that the amplitude of the pressure signal matches the blood pressure value (e.g. the brachial blood pressure), processing that is performed to reduce or remove noise in each pressure signal, and so on.

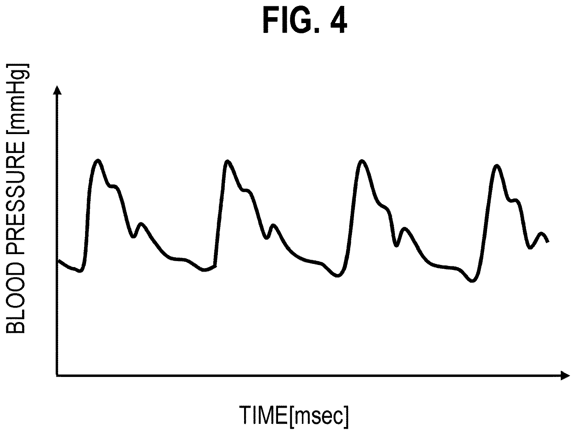

FIG. 4 shows a blood pressure waveform measured by the blood pressure measurement unit 20. The horizontal axis indicates time and the vertical axis indicates blood pressure. Although the sampling frequency may be set to any value, it is preferably set to be no less than 100 Hz so that characteristics of the shape of a waveform corresponding to one heartbeat can be reproduced. Typically, the period of one heartbeat is approximately one second, and therefore approximately one hundred or more data points can be acquired on a waveform corresponding to one heartbeat.

The blood pressure measurement unit 20 according to the present embodiment is advantageous in terms of the following.

The blood pressure measurement unit 20 can measure a blood pressure waveform for each heartbeat. As a result, it is possible to acquire various indicators related to blood pressure, the state of the heart, cardiovascular risks, and so on, based on the characteristics of the shape of the blood pressure waveform. In addition, it is possible to monitor for instantaneous values of blood pressure. Therefore, it is possible to instantaneously detect a blood pressure surge (a sudden rise in the blood pressure value), and to detect changes in blood pressure and irregularities in a blood pressure waveform that may occur in a very short period of time (corresponding to one to several heartbeats) without missing them.

As a portable blood pressure meter, a blood pressure meter that is to be worn on a wrist or an upper arm and employs an oscillometric method to measure blood pressure has come into practical use. However, a conventional portable blood pressure meter can only measure the mean value of blood pressure based on changes in the internal pressure of a cuff during a period of several seconds to a dozen or so seconds corresponding to a plurality of heartbeats, and cannot acquire time-series data regarding a blood pressure waveform for each heartbeat, unlike the blood pressure measurement unit 20 according to the present embodiment.

The blood pressure measurement unit 20 can record time-series data regarding blood pressure waveforms. By acquiring time-series data regarding blood pressure waveforms, and, for example, discerning characteristics of the blood pressure waveform related to temporal changes, or performing a frequency analysis on the time-series data to extract a specific frequency component, it is possible to acquire various indicators related to blood pressure, the state of the heart, cardiovascular risks, and so on.

The device employs a portable (wearable) type configuration, and less burden is placed on the user during measurement. Therefore, continuous measurement for a long time, and even 24-hour blood pressure monitoring, can be relatively easily performed. Also, since the device is of a portable type, changes in not only blood pressure under resting conditions, but also an ambulatory blood pressure (for example, during daily life or exercise) can be measured. As a result, it is possible to grasp how blood pressure is affected by behaviours in daily life (such as sleeping, eating, commuting, working, and taking medicine) and exercise, for example.

Conventional products are types of devices that measure blood pressure under resting conditions, with an arm or a wrist fixed to a blood pressure measurement unit, and cannot measure changes in blood pressure in daily life or during exercise, unlike the biological information analysis system 10 according to the present embodiment.

The blood pressure measurement unit 20 can be easily combined or linked with other sensors. For example, it is possible to make an evaluation of a cause-effect relationship or a composite evaluation with information that can be acquired by other sensors (e.g. a body movement, environmental information such as an atmospheric temperature, biological information such as SpO₂ and respiration information).

### Biological Information Analysis Device

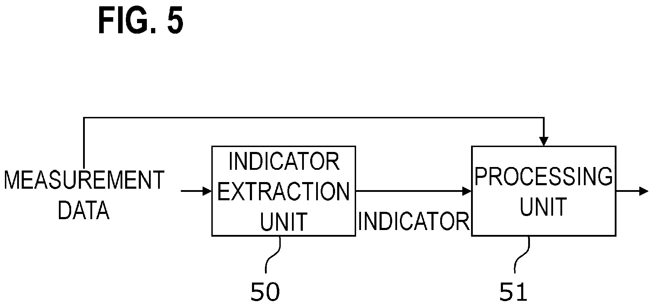

FIG. 5 is a block diagram illustrating processing that is performed by the biological information analysis device 1. As shown in FIG. 5, the biological information analysis device 1 includes an indicator extraction unit 50 and a processing unit 51. In the present embodiment, processing performed by the indicator extraction unit 50 and the processing unit 51 may be realized by the control unit 23 executing a program that is required for the processing. The program may be stored in the storage unit 27. When the control unit 23 executes the required program, the subject program stored in the ROM or storage unit 27 is loaded to the RAM. Then, the control unit 23 interprets and executes the program loaded to the RAM, using the CPU, to control each constituent component. Note that at least one or all of the processing procedures executed by the indicator extraction unit 50 and the processing unit 51 may be realized using a circuit such as an ASIC or an FPGA. Alternatively, at least one or all of the processing procedures executed by the indicator extraction unit 50 and the processing unit 51 may be realized using a computer (e.g. a smartphone, a tablet terminal, a personal computer, or a cloud server) that is separate from the main body 11.

The indicator extraction unit 50 acquires time-series data regarding blood pressure waveforms, which have been consecutively measured by the blood pressure measurement unit 20, from the storage unit 27. The indicator extraction unit 50 extracts, from the acquired time-series data regarding blood pressure waveforms, indicators that are related to characteristics of the blood pressure waveforms. Here, characteristics of a blood pressure waveform include, for example, characteristics of the shape of a blood pressure waveform corresponding to one heartbeat, temporal changes in a blood pressure waveform, and frequency components of a blood pressure waveform. However, characteristics of a blood pressure waveform are not limited to those listed above. The extracted indicators are output to the processing unit 51. There are various characteristics and indicators regarding a blood pressure waveform, and the characteristics and indicators that are to be extracted may be designed or selected as appropriate according to the purpose of processing that is to be performed by the processing unit 51. Characteristics and indicators that can be extracted from measurement data regarding blood pressure waveforms according to the present embodiment will be described later in detail.

When obtaining indicators, the indicator extraction unit 50 may use measurement data that has been acquired by the body movement measurement unit 21 and/or measurement data that has been acquired by the environment measurement unit 22, in addition to measurement data regarding blood pressure waveforms. Also, although not shown in the drawings, pieces of measurement data that have been acquired by a sleep sensor, an SpO₂ sensor, a blood-sugar level sensor, and the like may be combined with one another. By performing complex analysis on a plurality of kinds of measurement data acquired by a plurality of sensors, it is possible to perform more advanced information analysis of a blood pressure waveform. For example, it is possible to classify pieces of data regarding blood pressure waveforms according to states of the user, such as a resting state and a moving state, a state when an atmospheric temperature is high and a state when it is low, a light sleep state and a deep sleep state, a breathing state and an apnea state, and so on. Alternatively, it is possible to extract information regarding the influence of body movement, an activity amount, activity intensity, a change in an atmospheric temperature, apnea, the user's breathing, etc. on blood pressure, and thus evaluate the cause-effect relationship, the correlation, etc. between pieces of measurement data.

The processing unit 51 receives the indicators extracted by the indicator extraction unit 50. The processing unit 51 performs processing that is based on the received indicators. Various kinds of processing can be conceived of as processing that is based on the indicators. For example, the processing unit 51 may provide the values of the extracted indicators or changes in the values to a user, a doctor, a public health nurse, or the like to prompt the utilization of the indicators in the fields of health care, treatment, health guidance, and so on. Alternatively, the processing unit 51 may estimate cardiovascular risks from the extracted indicators, or provide guidelines for health maintenance or risk mitigation. Furthermore, when an increase in the risk of a cardiac disease occurring is detected or predicted based on an indicator, the processing unit 51 may inform the user or his/her doctor, or perform control to prevent the user from performing an action that burdens his/her heart and so on, or to prevent a cardiovascular event from occurring.

### Information Acquired from Blood Pressure Waveform

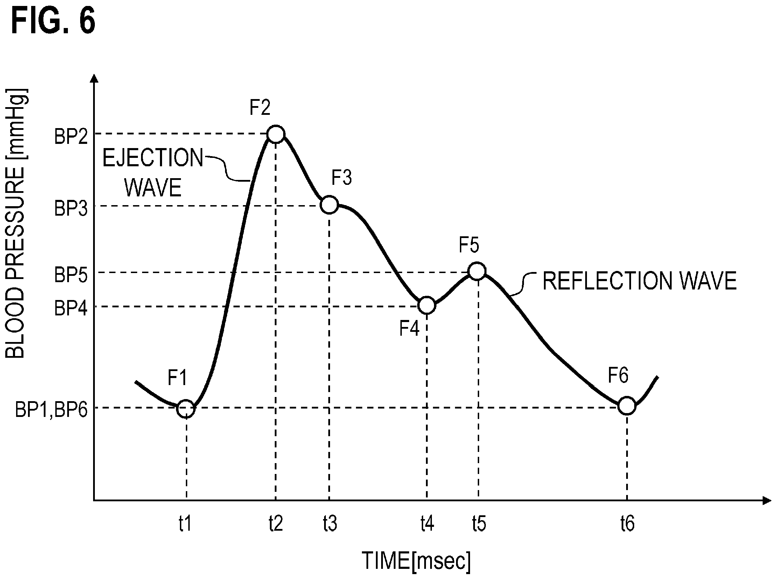

FIG. 6 shows a waveform (a blood pressure waveform) of a pressure pulse wave from a radial artery corresponding to one heartbeat. The horizontal axis indicates time t (msec) and the vertical axis indicates blood pressure BP (mmHg).

A blood pressure waveform is the waveform of a composite wave constituted by an "ejection wave" that is generated when the heart contracts and pumps out blood, and a "reflection wave" that is generated when an ejection wave is reflected at a branch point of a peripheral vessel or an artery. The following shows examples of characteristic points that can be extracted from a blood pressure waveform corresponding to one heartbeat.
- A point F1 is the rising point of the pressure pulse wave. The point F1 corresponds to the ejection start point of the heart, i.e. the point at which the aortic valve opens.
- A point F2 is a point at which the amplitude (the pressure) of the ejection wave is at the maximum (a first peak).
- A point F3 is an inflection point that appears midway in a drop in the ejection wave, due to a reflection wave being superimposed.
- A point F4 is the minimum point, which appears between the ejection wave and the reflection wave, and is also referred to as a notch. This point corresponds to the point at which the aortic valve closes.
- A point F5 is the peak of the reflection wave (a second peak), which appears after the point F4.
- A point F6 is the end point of one heartbeat, and corresponds to the ejection start point of the next heartbeat, i.e. the start point of the next heartbeat.

The indicator extraction unit 50 may use any algorithm to detect the above-described characteristic points. For example, the indicator extraction unit 50 may perform computations to obtain an nth order differential waveform of a blood pressure waveform, and detect the zero-crossing points to extract the characteristic points (the inflection points) of the blood pressure waveform (the points F1, F2, F4, F5, and F6 can be detected from the first order differential waveform, and the point F3 can be detected from the second order differential waveform or the fourth order differential waveform). Alternatively, the indicator extraction unit 50 may read out, from the storage unit 27, a waveform pattern on which the characteristic points have been arranged in advance, and perform fitting of the waveform pattern to the target blood pressure waveform to specify the respective positions of the characteristic points.

The indicator extraction unit 50 performs computations based on time t and pressure BP of each of the above-described characteristic points F1 to F6, and can thus obtain various kinds of information (values, characteristic amounts, indicators, etc.) from the blood pressure waveform of one heartbeat. The following are typical examples of information that can be acquired from a blood pressure waveform. Note that tx and BPx respectively represent time and blood pressure corresponding to a characteristic point Fx.
- Pulse Wave Interval (Period of Heartbeat) TA = t6 - t1
- Heart Rate PR = 1/TA
- Pulse Wave Rising Time UT = t2 - t1
- Systole TS = t4 - t1
- Diastole TD = t6 - t4
- Reflection Wave Delay Time = t3 - t1
- Maximum Blood Pressure (Systolic Blood Pressure) SBP = BP2
- Minimum Blood Pressure (Diastolic Blood Pressure) DBP = BP1
- Mean Blood Pressure MAP = (Area of Blood Pressure Waveform from t1 to t6)/Period of Heartbeat TA
- Mean Blood Pressure during Systole = (Area of Blood Pressure Waveform from t1 to t4)/Systole TS
- Mean Blood Pressure during Diastole = (Area of Blood Pressure Waveform from t4 to t6)/Diastole TD
- Pulse Pressure PP = Maximum Blood Pressure SBP - Minimum Blood Pressure DBP
- Late Systolic Pressure SBP2 = BP3
- AI (Augmentation Index) = (Late Systolic Pressure SBP2 - Minimum Blood Pressure DBP)/Pulse Pressure PP

Basic statistics of these pieces of information (values, characteristic amounts, and indicators) can also be used as indicators. Basic statistics include, for example, representative values (a mean value, a median value, a mode value, the maximum value, the minimum value, and so on) and the degree of scatter (dispersion, a standard deviation, a coefficient of variation, and so on). Temporal changes in these pieces of information (values, characteristic values, and indicators) can also be used as indicators.

In addition, the indicator extraction unit 50 can also acquire an indicator called BRS (Baroreflex Sensitivity) by performing computations on pieces of heartbeat information. This indicator indicates the ability to regulate blood pressure to be constant. Examples of methods for calculating the indicator include a spontaneous sequence method. This is a method for only extracting a sequence in which the maximum blood pressure SBP and the pulse wave interval TA consecutively rise or fall over the period of three or more heartbeats in synchronization with each other, plotting the maximum blood pressure SBP and the pulse wave interval TA onto a two-dimensional plane, and defining the inclination of the regression line obtained through a least squares method as the BRS.

As described above, the use of the biological information analysis system 10 according to the present embodiment makes it is possible to acquire various kinds of information from blood pressure waveform data. However, the biological information analysis system 10 need not implement all of the functions that are required to acquire all of the kinds of information described above. The biological information analysis system 10 need only implement functions that are required to acquire necessary information, depending on the configuration of the biological information analysis system 10, who the user is, the purpose of use, the location of use, and so on. Also, each function may be provided as a program module (a piece of application software), and the biological information analysis system 10 may employ a mechanism with which a function can be added by installing a necessary program module on the biological information analysis system 10.

The following illustrates an example, which is a specific application, of the biological information analysis system 10.

### Example 1

The present example proposes a method for suggesting a breathing technique that is suitable for the characteristics and the state of disease of each individual user in an objective manner by representing the relationship between breaths and changes in blood pressure based on time-series data regarding breathing and blood pressure measured from users.

Note that conventional blood pressure meters that employ an oscillometric method cannot accurately keep track of each breathing action, or the influence of each action of exhaling/inhaling on blood pressure, because a plurality of breathing actions are performed while blood pressure is measured once. In contrast, the device according to the present example can accurately and non-invasively measure a blood pressure waveform for each heartbeat, and is thus able to quantitatively analyze the influence of each action of exhaling/inhaling on blood pressure or the waveform thereof.

As shown in FIG. 2, the biological information analysis system 10 according to the present example includes a respiration sensor that serves as the respiration measurement unit 28. However, since it is only necessary to measure breathing and blood pressure waveforms in synchronization, the biological information analysis system 10 may not be provided with a respiration measurement unit, and the biological information analysis system 10 may simply use data measured by another respiration sensor. If this is the case, measurement data regarding blood pressure waveforms and measurement data acquired by the respiration sensor can be associated with each other in terms of time, based on measurement time information (time stamps), for example.

As a respiration sensor, a flow sensor that can detect the direction in which air flows, such as that disclosed in JP H10-185639A, can be favorably used. This flow sensor detects an air flow caused by the breathing action to determine whether the action is exhalation or inhalation. Alternatively, a pressure sensor or a vibration sensor may be used as a respiration sensor. If this is the case, body movement caused by the breathing action is detected by a pressure sensor, a vibration sensor, or the like that is attached to a body part, and thus the breathing action is indirectly detected.

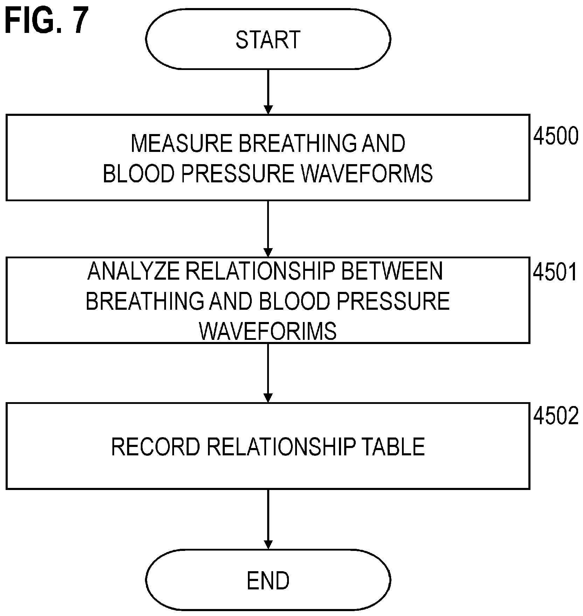

FIG. 7 shows an example of a flowchart for processing according to the present example. First, in order to grasp the relationship between breathing and changes in blood pressure, the user wears the biological information analysis system 10 and the respiration sensor, and blood pressure waveforms and breathing are measured (step 4500). Time-series data regarding blood pressure waveforms and time-series data regarding breathing are stored in the storage unit 27. The time-series data regarding breathing includes pieces of information regarding exhalation periods (periods of time during which air is exhaled) and pieces of information regarding inhalation periods (periods of time during which air is inhaled), which are arranged one after the other. A piece of information regarding an exhalation period includes the start time and the end time of the period, the length of the period (also referred to as the duration of exhalation), and the amount of breath in the period (the amount of exhaled air, which is also referred to as an exhale amount). A piece of information regarding an inhalation period includes the start time and the end time of the period, the length of the period (also referred to as the duration of inhalation), and the amount of breath in the period (the amount of inhaled air, which is also referred to as an inhale amount).

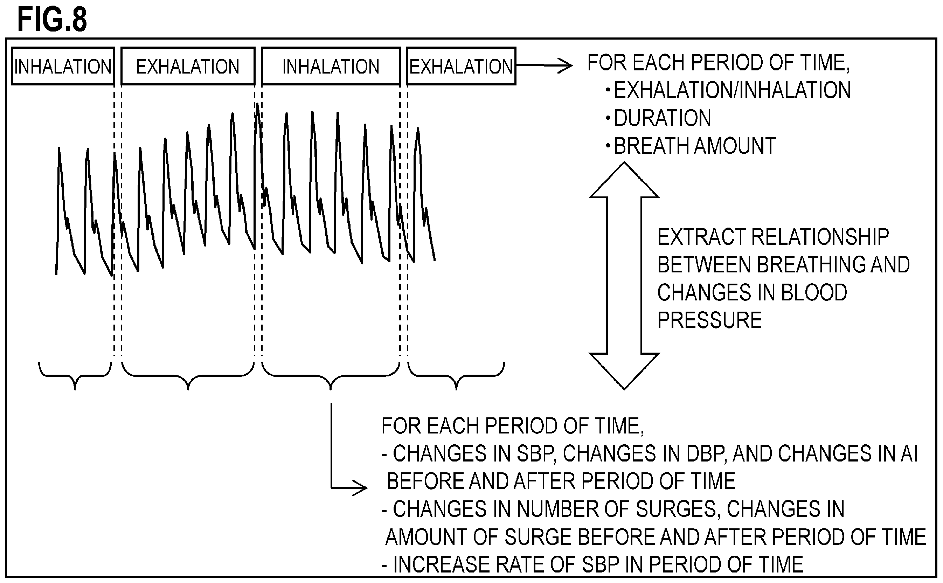

Next, the indicator extraction unit 50 analyzes the relationship between breathing and changes in blood pressure (step 4501). Specifically, the indicator extraction unit 50 reads, from the storage unit 27, time-series data regarding blood pressure waveforms, and time-series data regarding breathing measured in the period corresponding to the time-series data regarding blood pressure waveforms. Thereafter, as shown in FIG. 8, the indicator extraction unit 50 specifies exhalation periods and inhalation periods based on the time-series data regarding breathing, and divides the time-series data regarding blood pressure waveforms into blood pressure waveforms respectively corresponding to the periods. Thereafter, the indicator extraction unit 50 performs data mining processing such as cross tabulation or regression analysis on pieces of information regarding breathing respectively corresponding to the periods, and information regarding blood pressure waveforms corresponding thereto, to extract an indicator indicating the relationship between breathing and changes in blood pressure.

For example, the indicator extraction unit 50 classifies (typifies) the ways the user breathes, into a plurality of patterns, based on information regarding breathing in each period. The classification into breathing patterns may be performed based on, for example, the length of the period and/or the amount of breath. Here, exhalation periods and inhalation periods may be classified so as to be separate from each other, or classified into sets each consisting of an exhalation period and an inhalation period (each set is referred to as a breathing period). Thereafter, the indicator extraction unit 50 may collect pieces of data regarding blood pressure waveforms for a plurality of periods that have the same breathing pattern, and extract, as indicators indicating the relationship between breathing and changes in blood pressure, a trend in changes in blood pressure (an increase, a decrease, no change, and so on) and/or the amount of changes (the amount of an increase, the amount of a decrease, and so on) that are/is common to the pieces of data regarding blood pressure waveforms. By performing the same processing for each breathing pattern, it is possible to obtain an indicator indicating the relationship between breathing and changes in blood pressure for each of a plurality of breathing patterns.

Here, "data regarding blood pressure waveforms corresponding to a given period" may be "data regarding blood pressure waveforms in the given period" or "data regarding blood pressure waveforms of a plurality of periods that include the period and one or more periods that are previous and/or subsequent to the period". In the former case, changes in blood pressure waveforms within one period (for example, an increase rate and a decrease rate of systolic blood pressure) are items that are to be used to evaluate changes in blood pressure. In the latter case, changes in blood pressure waveforms within a plurality of periods (for example, the amount of change in systolic blood pressure (SBP), the amount of change in diastolic blood pressure (DBP), the amount of change in AI (Augmentation Index), the amount of change in the number of times a surge in blood pressure has occurred, and the amount of change in the amount of increase in a surge in blood pressure) are items that are to be used to evaluate changes in blood pressure. Alternatively, as "data regarding blood pressure waveforms corresponding to a given period", "data regarding blood pressure waveforms measured upon a predetermined period of time elapsing after the period" may be used. Such an analysis is effective if the influence of breathing on blood pressure waveforms appears upon a predetermined period of time elapsing.

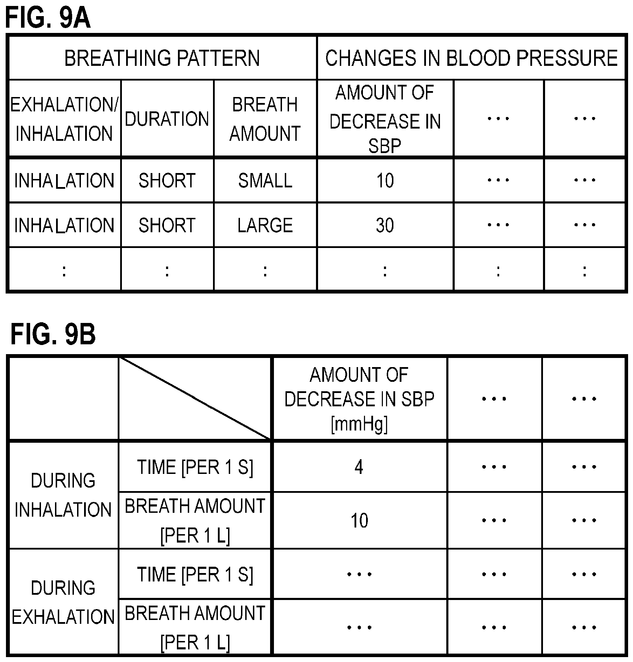

Next, the indicator extraction unit 50 stores the relationship between breathing and changes in blood pressure (an indicator) acquired in step 4501 for each breathing pattern, in the storage unit 27, in the form of a relationship table (step 4502). FIGS. 9A and 9B show an example of the relationship table. It can be seen from the relationship table shown in FIG. 9A that, if a small amount of air is inhaled in a short period of time, the SBP decreases by 10 mmHg, and if a large amount of air is inhaled even in a short period of time, the SBP decreases by 30 mmHg. Here, whether the duration of a period is long or short may be determined based on whether or not the duration of the period is longer than a threshold value. The threshold value is stored in the storage unit 27, for example. Similarly, whether the amount of breath is large or small may be determined based on whether or not the amount of breath in the period is greater than a threshold value. The threshold values may be fixed, or changed as appropriate according to the tendencies and the characteristics of the user. It can be seen from the relationship table shown in FIG. 9B that the SBP decreases by 4 mmHg per second during inhalation, and the SBP decreases by 10 mmHg per liter of breath. Note that tables shown in FIGS. 9A and 9B are examples, and it is also preferable that the amount of changes in the DBP and the amount changes in the AI are recorded. It can be said that these relationship tables are indicators indicating the relationship between breathing and change in blood pressure. The relationship tables thus generated are stored in the storage unit 27, and used for various kinds of processing that is performed by the processing unit 51.

Note that, if it is possible to use a past case database in which relationship tables are registered, which show the relationship between breathing and changes in blood pressure for a large number of subjects, the indicator extraction unit 50 may acquire, from the past case database, data of another subject who is similar to the user in terms of the relationship between breathing and changes in blood pressure, and generate a relationship table for the user, considering the data of the subject as well. Thus, it is possible to generate a more accurate and objective relationship table.

Next, the following describes an example of processing that is performed by the processing unit 51. For example, the processing unit 51 may read a relationship table stored in the storage unit 27, and output information representing the relationship between the user's breathing and changes in blood pressure, from the output unit 25. At this time, information representing the relationship between the user's breathing and changes in blood pressure may be output in the form of a table as shown in FIGS. 9A and 9B, or in a different form.

Also, the processing unit 51 may perform breathing pattern recommendation processing based on a relationship table. Breathing pattern recommendation processing is performed to suggest a breathing pattern that is to be followed in order to achieve a desired change in blood pressure. For example, it is envisioned that a user experiences an increase in blood pressure or poor physical condition, and wishes to regulate blood pressure by using a breathing technique. If this is the case, the user inputs a target value of a change in blood pressure, using the input unit 24 of the biological information analysis system 10. For example, it is envisioned that that user has entered a target value to "lower the systolic blood pressure (SBP) by 30 mmHg". The processing unit 51 refers to relationship tables in the storage unit 27, and designs a breathing pattern (the duration of exhalation, the duration of inhalation, an exhale amount, an inhale amount, the number of breaths, and so on) that is required to lower the SBP by 30 mmHg. At this time, if a plurality of kinds of breathing patterns can be conceived of, the most appropriate breathing pattern is preferably selected, considering the ease with which the user can follow the pattern.

Thereafter, the processing unit 51 recommends a desirable breathing pattern to the user via the output unit 25. For example, it is preferable that the processing unit 51 recommends a specific breathing pattern, saying "breathe in for five or more seconds, ten times", for example. As a result, the user can control his/her blood pressure, using an appropriate breathing technique, and prevent a cardiovascular event from occurring.

However, if the user is recommended to "breathe in for five or more seconds, ten times", there is the possibility of the user being unable to actually breathe as instructed. Therefore, the processing unit 51 may provide a breathing exercise function. For example, in a state where the respiration sensor and the biological information analysis system 10 are worn, the processing unit 51 provides a task, saying "breathe in for five or more seconds, ten times", for example, from the output unit 25. While the user is performing the task, the processing unit 51 monitors breathing and blood pressure, using the respiration sensor and the biological information analysis system 10, and evaluates whether or not the user is successfully breathing and controlling his/her blood pressure according to the task, and notifies the user of the results of evaluation via the output unit 25. Using such a function, the user can acquire a breathing technique in an objective manner.

The configurations according to the above-described embodiment and examples are no more than specific examples of configurations according to the present invention, and are not intended to limit the scope of the present invention, which is defined by the appended claims.

### INDEX TO THE REFERENCE NUMERALS

1 ... biological information analysis device, 2 ... measurement unit
10 ... biological information analysis system, 11 ... main body, 12 ... belt
20 ... blood pressure measurement unit, 21 ... body movement measurement unit, 22 ... environment measurement unit, 23 ... control unit, 24 ... input unit, 25 ... output unit, 26 ... communication unit, 27 ... storage unit, 28 ... respiration measurement unit
30 ... pressure sensor, 31 ... pressurizing mechanism, 300 ... pressure detection element
50 ... indicator extraction unit, 51 ... processing unit

## Claims

1. A biological information analysis device (1) comprising:
an indicator extraction unit (50) configured to
extract, from time-series data regarding blood pressure waveforms consecutively measured by a sensor (30) that is configured to be worn on a body part of a user and to be capable of non-invasively measuring a blood pressure waveform for each heartbeat, and from time-series data regarding breathing measured by a respiration sensor during a period of time corresponding to the time-series data regarding blood pressure waveforms, an indicator indicating a relationship between the user's breathing and changes in blood pressure,
specify a plurality of periods of time that have the same breathing pattern, from the time-series data regarding breathing,
extract an indicator indicating the relationship between breathing and changes in blood pressure for the breathing pattern, from pieces of data regarding blood pressure waveforms respectively corresponding to the plurality of periods of time,
classify ways the user breathes, into a plurality of breathing patterns, based on the time-series data regarding the user's breathing,
extract an indicator indicating the relationship between breathing and changes in blood pressure for each of the plurality of breathing patterns, and
generate, for each of the plurality of breathing patterns, a relationship table that defines an indicator indicating the relationship between breathing and changes in blood pressure; and
a processing unit (51) configured to
perform processing that is based on the indicator thus extracted, and
select a breathing pattern that is to be followed in order to achieve a desired change in blood pressure, based on the relationship table, and
recommend the selected breathing pattern to the user.

2. The biological information analysis device according (1) to claim 1,
wherein the indicator extraction unit (50) is configured to extract, as the indicator, a tendency in changes in blood pressure and/or the amount of changes that is/are common to pieces of data regarding blood pressure waveforms.

3. The biological information analysis device (1) according to claim 1 or 2,
wherein the time-series data regarding breathing includes, for each of a plurality of periods of time that include an exhalation period and an inhalation period, the length of the period of time and/or the amount of breath, and
the indicator extraction unit (50) is configured to classify the plurality of periods of time into a plurality of breathing patterns based on the length of the period of time and/or the amount of breath.

4. The biological information analysis device (1) according to any one of claims 1 to 3,
wherein the processing unit (51) is configured to perform processing to output information representing the relationship between the user's breathing and changes in blood pressure, based on the indicator.

5. The biological information analysis device (1) according to any one of claims 1 to 4,
wherein the indicator extraction unit (50) includes, as changes in blood pressure, at least one of: changes in systolic blood pressure; changes in diastolic blood pressure; changes in an AI (Augmentation Index); changes in the number of times a surge in blood pressure occurs; and changes in the amount of an increase in a surge in blood pressure.

6. A biological information analysis system (10) comprising:
a sensor that is configured to be worn on a body part of a user and to be capable of non-invasively measuring a blood pressure waveform for each heartbeat; and
the biological information analysis device (1) according to any one of claims 1 to 5, the biological information analysis device (1) being configured to analyze biological information, using data regarding blood pressure waveforms consecutively measured by the sensor (30).

7. The biological information analysis system (10) according to claim 6, further comprising:
a respiration sensor.

8. A program that causes a processor to function as the indicator extraction unit (50) and the processing unit (51) of the biological information analysis device (1) according to any one of claims 1 to 5.

9. A biological information analysis method comprising the following steps performed by an extraction unit (50):
extracting, from time-series data regarding blood pressure waveforms consecutively measured by a sensor (30) that is configured to be worn on a body part of a user and to be time-series data regarding breathing measured by a respiration sensor during a period of time corresponding to the time-series data regarding blood pressure waveforms, an indicator indicating a relationship between the user's breathing and changes in blood pressure;
specifying a plurality of periods of time that have the same breathing pattern, from the time-series data regarding breathing,
extracting an indicator indicating the relationship between breathing and changes in blood pressure for the breathing pattern, from pieces of data regarding blood pressure waveforms respectively corresponding to the plurality of periods of time;
classifying ways the user breathes, into a plurality of breathing patterns, based on the time-series data regarding the user's breathing;
extracting an indicator indicating the relationship between breathing and changes in blood pressure for each of the plurality of breathing patterns;
generating, for each of the plurality of breathing patterns, a relationship table that defines an indicator indicating the relationship between breathing and changes in blood pressure; the method further comprising the following steps performed by a processing unit (51):
performing processing that is based on the indicator thus extracted;
selecting a breathing pattern that is to be followed in order to achieve a desired change in blood pressure, based on the relationship table; and
recommending the selected breathing pattern to the user.

## Patentansprüche

1. Vorrichtung (1) zur Analyse biologischer Informationen, umfassend:
eine Indikator-Extraktionseinheit (50), die eingerichtet ist zum:
Extrahieren, aus Zeitreihendaten bezüglich Blutdruckwellenformen, die aufeinanderfolgend durch einen Sensor (30) gemessen werden, der dafür eingerichtet ist, an einem Körperteil eines Benutzers getragen zu werden und auf nicht-invasive Weise eine Blutdruckwellenform für jeden Herzschlag messen zu können, sowie aus Zeitreihendaten bezüglich einer Atmung, die durch einen Atmungssensor während eines Zeitraums gemessen werden, die den Zeitreihendaten bezüglich Blutdruckwellenformen entspricht,
eines Indikators, der eine Beziehung zwischen der Atmung des Benutzers und Blutdruckänderungen anzeigt,
Spezifizieren mehrerer Zeiträume, die das gleiche Atmungsmuster aufweisen, aus den Zeitreihendaten bezüglich der Atmung,
Extrahieren eines Indikators, der die Beziehung zwischen Atmung und Blutdruckänderungen für das Atmungsmuster anzeigt, aus Daten bezüglich Blutdruckwellenformen, die jeweils den mehreren Zeiträumen entsprechen,
Klassifizieren der Art und Weise, wie der Benutzer atmet, in mehrere Atmungsmuster anhand der Zeitreihendaten bezüglich der Atmung des Benutzers,
Extrahieren eines Indikators, der die Beziehung zwischen Atmung und Blutdruckänderungen für jedes der mehreren Atmungsmuster anzeigt, und
Generieren, für jedes der mehreren Atmungsmuster, einer Beziehungstabelle, die einen Indikator definiert, der die Beziehung zwischen Atmung und Blutdruckänderungen anzeigt, und
eine Verarbeitungseinheit (51), die eingerichtet ist zum:
Ausführen einer Verarbeitung, die auf dem in dieser Weise extrahierten Indikator basiert, und
Auswählen eines Atmungsmusters, das befolgt werden soll, um eine gewünschte Blutdruckänderung zu erreichen, auf der Grundlage der Beziehungstabelle, und
Empfehlen des ausgewählten Atmungsmusters an den Benutzer.

2. Vorrichtung (1) zur Analyse biologischer Informationen nach Anspruch 1,
wobei die Indikator-Extraktionseinheit (50) dafür eingerichtet ist, als den Indikator eine Tendenz der Blutdruckänderungen und/oder den Betrag von Änderungen zu extrahieren, die bzw. der den Daten bezüglich der Blutdruckwellenformen gemein sind.

3. Vorrichtung (1) zur Analyse biologischer Informationen nach Anspruch 1 oder 2,
wobei die Zeitreihendaten bezüglich der Atmung für jeden von mehreren Zeiträumen, die einen Ausatmungszeitraum und einen Einatmungszeitraum umfassen, die Länge des Zeitraums und/oder die Atemmenge umfassen, und
die Indikator-Extraktionseinheit (50) dafür eingerichtet ist, die mehreren Zeiträume auf der Grundlage der Länge des Zeitraums und/oder der Atemmenge in mehrere Atmungsmuster zu klassifizieren.

4. Vorrichtung (1) zur Analyse biologischer Informationen nach einem der Ansprüche 1 bis 3, wobei die Verarbeitungseinheit (51) dafür eingerichtet ist, auf der Grundlage des Indikators eine Verarbeitung durchzuführen, um Informationen auszugeben, die die Beziehung zwischen der Atmung des Benutzers und Blutdruckänderungen darstellen.

5. Vorrichtung (1) zur Analyse biologischer Informationen nach einem der Ansprüche 1 bis 4,
wobei die Indikator-Extraktionseinheit (50) als Blutdruckänderungen mindestens eines von Folgendem umfasst: Änderungen des systolischen Blutdrucks, Änderungen des diastolischen Blutdrucks, Änderungen eines AI (Augmentierungs-Index), Änderungen der Anzahl des Auftretens eines Blutdruckanstiegs, und Änderungen des Betrages einer Zunahme eines Blutdruckanstiegs.

6. System (10) zur Analyse biologischer Informationen, umfassend:
einen Sensor, der dafür eingerichtet ist, an einem Körperteil eines Benutzers getragen zu werden und in nichtinvasiver Weise eine Blutdruckwellenform für jeden Herzschlag messen zu können, und
die Vorrichtung (1) zur Analyse biologischer Informationen nach einem der Ansprüche 1 bis 5, wobei die Vorrichtung (1) zur Analyse biologischer Informationen dafür eingerichtet ist, biologische Informationen unter Verwendung von Daten bezüglich Blutdruckwellenformen zu analysieren, die aufeinanderfolgend durch den Sensor (30) gemessen werden.

7. System (10) zur Analyse biologischer Informationen nach Anspruch 6, des Weiteren umfassen:
einen Atmungssensor.

8. Programm, das einen Prozessor veranlasst, als die Indikatorextraktionseinheit (50) und die Verarbeitungseinheit (51) der Vorrichtung (1) zur Analyse biologischer Informationen nach einem der Ansprüche 1 bis 5 zu dienen.

9. Verfahren zur Analyse biologischer Informationen, umfassend die folgenden Schritte, die durch eine Extraktionseinheit (50) ausgeführt werden:
Extrahieren, aus Zeitreihendaten bezüglich Blutdruckwellenformen, die aufeinanderfolgend durch einen Sensor (30) gemessen werden, der dafür eingerichtet ist, an einem Körperteil eines Benutzers getragen zu werden und auf nicht-invasive Weise eine Blutdruckwellenform für jeden Herzschlag messen zu können, sowie aus Zeitreihendaten bezüglich einer Atmung, die durch einen Atmungssensor während eines Zeitraums gemessen werden, die den Zeitreihendaten bezüglich Blutdruckwellenformen entspricht, eines Indikators, der eine Beziehung zwischen der Atmung des Benutzers und Blutdruckänderungen anzeigt,
Spezifizieren mehrerer Zeiträume, die das gleiche Atmungsmuster aufweisen, aus den Zeitreihendaten bezüglich der Atmung,
Extrahieren eines Indikators, der die Beziehung zwischen Atmung und Blutdruckänderungen für das Atmungsmuster anzeigt, aus Daten bezüglich Blutdruckwellenformen, die jeweils den mehreren Zeiträumen entsprechen,
Klassifizieren der Art und Weise, wie der Benutzer atmet, in mehrere Atmungsmuster anhand der Zeitreihendaten bezüglich der Atmung des Benutzers,
Extrahieren eines Indikators, der die Beziehung zwischen Atmung und Blutdruckänderungen für jedes der mehreren Atmungsmuster anzeigt, und
Generieren, für jedes der mehreren Atmungsmuster, einer Beziehungstabelle, die einen Indikator definiert, der die Beziehung zwischen Atmung und Blutdruckänderungen anzeigt, wobei das Verfahren des Weiteren den folgenden Schritt umfasst, der durch eine Verarbeitungseinheit (51) ausgeführt wird:
Ausführen einer Verarbeitung, die auf dem in dieser Weise extrahierten Indikator basiert,
Auswählen eines Atmungsmusters, das befolgt werden soll, um eine gewünschte Blutdruckänderung zu erreichen, auf der Grundlage der Beziehungstabelle, und
Empfehlen des ausgewählten Atmungsmusters an den Benutzer.

## Revendications

1. Dispositif d'analyse d'informations biologiques (1) comprenant :
une unité d'extraction d'indicateur (50) conçue pour :
extraire, de données de séries chronologiques concernant des formes d'onde de pression artérielle mesurées successivement par un capteur (30) qui est conçu pour être porté sur une partie du corps d'un utilisateur et pour être apte à mesurer de façon non effractive une forme d'onde de pression artérielle pour chaque battement de cœur, et de données de séries chronologiques concernant la respiration mesurée par un capteur de respiration au cours d'une période de temps correspondant aux données de séries chronologiques concernant des formes d'onde de pression artérielle, un indicateur indiquant une relation entre la respiration de l'utilisateur et des variations de pression artérielle,
spécifier une pluralité de périodes de temps qui ont le même mode respiratoire, à partir des données de séries chronologiques concernant la respiration,
extraire un indicateur indiquant la relation entre la respiration et des variations de pression artérielle pour le mode respiratoire, d'éléments de données concernant des formes d'onde de pression artérielle correspondant respectivement à la pluralité de périodes de temps,
classer les façons dont l'utilisateur respire, dans une pluralité de modes respiratoires, sur la base des données de séries chronologiques concernant la respiration de l'utilisateur,
extraire un indicateur indiquant la relation entre la respiration et des variations de pression artérielle pour chaque mode de la pluralité de modes respiratoires, et
créer, pour chaque mode de la pluralité de modes respiratoires, une table de relation qui définit un indicateur indiquant la relation entre la respiration et des variations de pression artérielle ; et
une unité de traitement (51) conçue pour :
effectuer un traitement qui est fondé sur l'indicateur ainsi extrait, et
sélectionner un mode respiratoire qui doit être suivi afin d'obtenir une modification souhaitée de la pression artérielle, sur la base de la table de relation, et recommander le mode respiratoire sélectionné à l'utilisateur.

2. Dispositif d'analyse d'informations biologiques (1) selon la revendication 1,
dans lequel l'unité d'extraction d'indicateur (50) est conçue pour extraire, en tant qu'indicateur, une tendance de variations de pression artérielle et/ou la grandeur de variations qui est/sont commune(s) à des éléments de données concernant des formes d'onde de pression artérielle.

3. Dispositif d'analyse d'informations biologiques (1) selon la revendication 1 ou 2,
dans lequel les données de séries chronologiques concernant la respiration incluent, pour chaque période d'une pluralité de périodes de temps qui incluent une période d'expiration et une période d'inspiration, la durée de la période de temps et/ou le volume respiratoire, et
l'unité d'extraction d'indicateur (50) est conçue pour classer la pluralité de périodes de temps dans une pluralité de modèles respiratoires en fonction de la durée de la période de temps et/ou du volume respiratoire.

4. Dispositif d'analyse d'informations biologiques (1) selon l'une quelconque des revendications 1 à 3,
dans lequel l'unité de traitement (51) est conçue pour effectuer un traitement afin de délivrer des informations représentant la relation entre la respiration de l'utilisateur et des variations de pression artérielle, sur la base de l'indicateur.

5. Dispositif d'analyse d'informations biologiques (1) selon l'une quelconque des revendications 1 à 4,
dans lequel l'unité d'extraction d'indicateur (50) inclut, en tant que variations de pression artérielle : des variations de pression artérielle systolique ; et/ou des variations de pression artérielle diastolique ; et/ou des variations de l'IA (index d'augmentation) ; et/ou des variations du nombre de fois que se produit un pic de pression artérielle ; et/ou des variations de la grandeur d'élévation d'un pic de pression artérielle.

6. Système d'analyse d'informations biologiques (10) comprenant :
un capteur qui est conçu pour être porté sur une partie du corps d'un utilisateur et pour être apte à mesurer de façon non effractive une forme d'onde de pression artérielle pour chaque battement de cœur ; et
le dispositif d'analyse d'informations biologiques (1) selon l'une quelconque des revendications 1 à 5, le dispositif d'analyse d'informations biologiques (1) étant conçu pour analyser des informations biologiques à l'aide de données concernant des formes d'onde de pression artérielle mesurées successivement par le capteur (30).

7. Système d'analyse d'informations biologiques (10) selon la revendication 6, comprenant en outre :
un capteur de respiration

8. Programme qui fait fonctionner un processeur comme l'unité d'extraction d'indicateur (50) et l'unité de traitement (51) du dispositif d'analyse d'informations biologiques (1) selon l'une quelconque des revendications 1 à 5.

9. Procédé d'analyse d'informations biologiques comprenant les étapes suivantes exécutées par une unité d'extraction (50) :
extraire, de données de séries chronologiques concernant des formes d'onde de pression artérielle mesurées successivement par un capteur (30) qui est conçu pour être porté sur une partie du corps d'un utilisateur et pour être apte à mesurer de façon non effractive une forme d'onde de pression artérielle pour chaque battement de cœur, et de données de séries chronologiques concernant la respiration mesurée par un capteur de respiration au cours d'une période de temps correspondant aux données de séries chronologiques concernant des formes d'onde de pression artérielle, un indicateur indiquant une relation entre la respiration de l'utilisateur et des variations de pression artérielle ;
spécifier une pluralité de périodes de temps qui ont le même mode respiratoire, à partir des données de séries chronologiques concernant la respiration ;
extraire un indicateur indiquant la relation entre la respiration et des variations de pression artérielle pour le mode respiratoire, d'éléments de données concernant des formes d'onde de pression artérielle correspondant respectivement à la pluralité de périodes de temps ;
classer les façons dont l'utilisateur respire, dans une pluralité de modes respiratoires, sur la base des données de séries chronologiques concernant la respiration de l'utilisateur ;
extraire un indicateur indiquant la relation entre la respiration et des variations de pression artérielle pour chaque mode de la pluralité de modes respiratoires ;
créer, pour chaque mode de la pluralité de modes respiratoires, une table de relation qui définit un indicateur indiquant la relation entre la respiration et des variations de pression artérielle ;
le procédé comprenant en outre les étapes suivantes exécutées par une unité de traitement (51) :
effectuer un traitement qui est fondé sur l'indicateur ainsi extrait ;
sélectionner un mode respiratoire qui doit être suivi afin d'obtenir une modification souhaitée de la pression artérielle, sur la base de la table de relation ; et
recommander le mode respiratoire sélectionné à l'utilisateur.
